# EUROPEAN PATENT SPECIFICATION

(11) **EP 2 545 065 B1**
(45) Date of publication and mention of the grant of the patent: **11.11.2015**
(21) Application number: 11754097.1
(22) Date of filing: 10.03.2011
(51) Int. Cl.: C07J 9/00, C07J 75/00, A61K 31/575, A61K 47/30, A61P 9/00

(54) **WATER-SOLUBLE PHYTOSTEROL DERIVATIVES FOR REDUCING CHOLESTEROL AND PREPARATION THEREOF**
WASSERLÖSLICHE PHYTOSTEROLDERIVATE ZUR CHOLESTERINSENKUNG UND HERSTELLUNG DAVON
DÉRIVÉS DE PHYTOSTÉROL HYDROSOLUBLES POUR ABAISSER LE CHOLESTÉROL ET PRÉPARATION DE CEUX-CI

(30) Priority: 12.03.2010 US 313421 P
(43) Date of publication of application: 16.01.2013
(60) Divisional application: 15180959.7
(73) Proprietor: KIP Biotech LLC, Minnetonka, MN 55305 (US)
(72) Inventor: CHATLA, Narayana, Mason, OH 45040 (US); RAMBACHER, Robert, William, Middletown, OH 45044 (US); YAO, Yao, Mason, OH 45040 (US)
(74) Representative: Grund, Martin
(86) International application number: PCT/US2011/027950
(87) International publication number: WO 2011/112840

(56) References cited:
- US-A- 3 004 043
- US-A- 5 298 611
- US-A- 6 045 826
- US-A1- 2008 070 981
- DATABASE CA [Online] CHEMICAL ABSTRACTS SERVICE, COLUMBUS, OHIO, US; 2007, CHUNG, DAE-WON ET AL: "Synthesis and solubility of hydrophilic derivatives of .beta.-", XP002714963, retrieved from STN Database accession no. 2007:627514 & CHUNG, DAE-WON ET AL: "Synthesis and solubility of hydrophilic derivatives of .beta.-", JOURNAL OF INDUSTRIAL AND ENGINEERING CHEMISTRY (SEOUL, REPUBLIC OF KOREA) , 13(3), 367-372 CODEN: JIECFI; ISSN: 1226-086X, 2007,

## Description

### TECHNICAL FIELD

The present disclosure relates to a method for preparing water-soluble phytosterol derivatives with cholesterol-reducing effects.

### BACKGROUND

β-sitosterol, (24-ethyl-5-cholestene-3-ol) and other phytosterols have been found to function as cholesterol lowering agents when used as food ingredients. Phytosterols such as β-sitosterol, compete directly with LDL-cholesterol in the body, reduce absorption of LDL-cholesterol in the intestines, and reduce serum cholesterol levels. Wide-spread application of such sterols as food ingredients is hindered by the hydrophobic and lipophilic nature of the compounds. These properties can limit the types of formulations that can be prepared, hindering the use of phytosterols as food ingredients.

### SUMMARY

Provided herein is a method of making a compound of Formula I:

X-L-P

or a salt form thereof, wherein: X is a phytosterol; L is a linker; and P is a water-soluble carrier selected from the group consisting of polyethylene glycol having an average molecular weight of about 200 to about 2000, polyalcohol, and polyether. In some embodiments, the method comprises: (a) reacting a mixture comprising X-L, P, an acid, and a first organic solvent, wherein the molar ratio of X-L to P is from about 1:1.5 to about 1:10; (b) neutralizing the mixture of step (a) and adding a second organic solvent; (c) washing the solution from step (b) with brine; and (d) isolating a composition comprising the compound of Formula I.

In some embodiments, the method further comprises recrystallizing the compound of Formula I.

The molar ratio of molar ratio of X-L to P can be from about 1:2 to about 1:6. For example, the molar ratio of X-L to P can be about 1:3.

The phytosterol (X) can be selected from the group consisting of: sitosterol, sitostanol, campesterol, campestanol, stigmasterol, stigmastanol, ergosterol, ergostanol, avenasterol, avenastanol, spinasterol, spinastanol, brassicasterol, brassicastanol, clionasterol, clionastanol and mixtures thereof. For example, the sitosterol can be β-sitosterol.

The linker (L) can be a dicarboxylate. For example, the linker can be selected from the group consisting of succinate, adipate, glutarate, pimelate, and malonate.

In some embodiments, P is polyethylene glycol having an average molecular weight of about 200 to about 2000. For example, P can have an average molecular weight of about 400 to about 1000.

In a method as described herein, the compound X-L, or a salt form thereof, can be prepared by a method comprising: (a) reacting a mixture of X and L in an organic solvent; (b) cooling the reaction mixture of step (a); (c) filtering the cooled reaction mixture of step (b); and (d) washing the filtrate with water and isolating a composition comprising the compound X-L. In some embodiments, the mixture of step (a) further comprises a base. For example, the base can be selected from the group consisting of DMAP, DIPEA, pyridine, and TEA. In some cases, the base is present in a catalytic amount.

An acid can be, for example, sulfuric acid, *p*-toluene sulfonic acid, or methanesulfonic acid. In some embodiments, the acid is present in a catalytic amount.

The organic solvents used herein can be independently selected from GRAS solvents. In some cases, the first solvent is toluene and the second solvent is ethyl acetate.

In some embodiments, the reacting comprises heating.

Also provided herein is a method of making a compound of Formula I, the method comprising reacting a mixture comprising X-L, P, and an acid, wherein the molar ratio of X-L to P is from about 1:1.5 to about 1:10.

Further provided herein is a method of purifying a compound of Formula I, the method comprising: (a) providing a solution comprising X-L-P and an organic solvent; (b) washing the solution with brine; and (c) isolating the compound of Formula I. A method of making a compound of Formula I is provided herein, the method comprising: (a) reacting a mixture of X and L in a first organic solvent; (b) cooling the reaction mixture of step (a); (c) filtering the cooled reaction mixture of step (b); (d) washing the filtrate with water and isolating a composition comprising the compound X-L; (e) reacting a mixture comprising X-L, P, an acid, and a second organic solvent, wherein the molar ratio of X-L to P is from about 1:1.5 to about 1:10; (f) neutralizing the mixture of step (e) and adding a third organic solvent; (g) washing the solution from step (f) with brine; and (h) isolating a composition comprising the compound of Formula I.

For example, provided herein is a method of making a compound of Formula III: 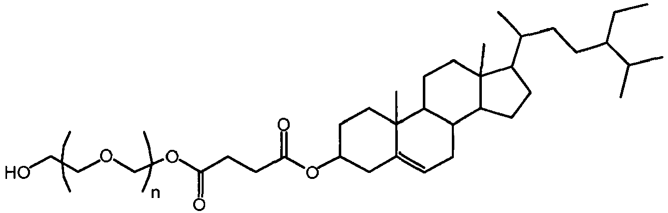 or a salt form thereof, the method comprising: (a) reacting a mixture comprising a compound of Formula IV: 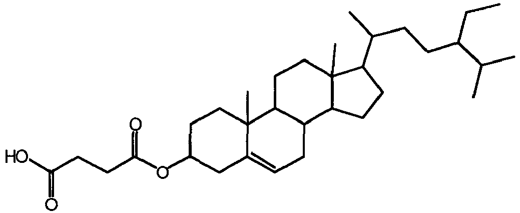 or a salt form thereof, polyethylene glycol having an average molecular weight of about 200 to about 2000, an acid, and a first organic solvent, wherein the molar ratio of the compound of Formula IV to polyethylene glycol is from about 1:1.5 to about 1:10; (b) neutralizing the mixture of step (a) and adding a second organic solvent; (c) washing the solution from step (b) with brine; and (d) isolating a composition comprising the compound of Formula III.

In some embodiments, the method described above can further comprise recrystallizing the compound of Formula III.

The molar ratio of the compound of Formula IV to polyethylene glycol can be from about 1:2 to about 1:6. For example, the molar ratio of the compound of Formula IV to polyethylene glycol can be about 1:3.

In some embodiments, the polyethylene glycol has an average molecular weight of about 400 to about 1000.

A method as described above can include the preparation of a compound of Formula IV, or a salt form thereof. The method can comprise: (a) reacting a mixture of β-sitosterol and succinic anhydride in an organic solvent; (b) cooling the reaction mixture of step (a); (c) filtering the cooled reaction mixture of step (b); and (d) washing the filtrate with water and isolating a composition comprising the compound of Formula IV.

Further provided herein is a method of making a compound of Formula III: 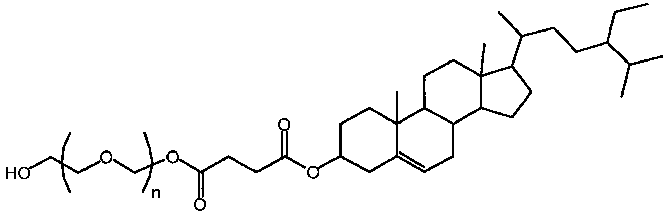 or a salt form thereof, the method comprising: (a) reacting a mixture of β-sitosterol and succinic anhydride in an organic solvent; (b) cooling the reaction mixture of step (a); (c) filtering the cooled reaction mixture of step (b); and (d) washing the filtrate with water and isolating a composition comprising a compound of Formula IV: 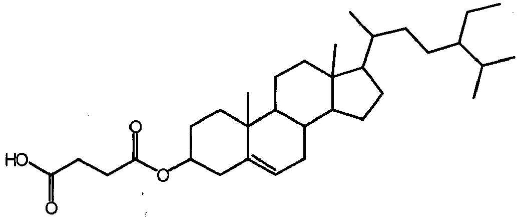 or a salt form thereof;
(e) reacting a mixture comprising the compound of Formula IV, or a salt form thereof, polyethylene glycol having an average molecular weight of about 200 to about 2000, an acid, and a second organic solvent, wherein the molar ratio of the compound of Formula IV to polyethylene glycol is from about 1:1.5 to about 1:10; (f) neutralizing the mixture of step (e) and adding a third organic solvent; (g) washing the solution from step (f) with brine; and (h) isolating a composition comprising the compound of Formula III.

A composition comprising a compound of Formula I or III prepared by the methods described herein can be soluble and/or dispersible in water in an amount of at least 30 mg of phytosterol per mL of water, and forms a clear dispersion. In some embodiments, a composition comprising a compound of Formula I or III prepared by the methods described herein can have a degree of substitution of about 1.0 to about 1.3.

The details of one or more embodiments of the invention are set forth in the accompanying drawings and the description below. Other features, objects, and advantages of the invention will be apparent from the description and drawings, and from the claims.

### DETAILED DESCRIPTION

Provided herein is a method for preparing phytosterol derivatives with higher solubility and safety compared to existing phytosterol compounds. Previous attempts to produce water-soluble derivatives have resulted in mixtures comprising phytosterols attached to both ends of the water-soluble carrier PEG ('di-type'), phytosterols attached to one end of PEG ('mono-type'), and unreacted PEG. See, for example, D. Chung and Y.T. Choi, J. Ind. Eng. Chem., 13(3): 367-372 (2007),US-A-3,004,043 and WO 2000/52029. The presence of the above di-type conjugate has been found to be a major cause of the limited solubility of these mixtures. The water-soluble phytosterol derivatives described herein use a low-molecular weight water-soluble carrier (e.g., low-molecular weight PEG) to increase the weight ratio of phytosterol in the derivatives and for ease of approval of the derivatives by the FDA as a food additive. The methods described herein function to control the synthesis of the derivatives and produce primarily the mono-type derivative through regulation of the mole ratio of the water-soluble carriers and phytosterols in the coupling reactions. The water-soluble phytosterol derivatives prepared using the methods described herein provide phytosterol derivatives which are safe, highly water-soluble, useful in both foods and drug applications, and can be applicable to the treatment of hypercholesterolemia, certain cardiac disorders, and hypertension.

### Water-soluble phytosterol derivatives

Water-soluble phytosterol derivatives include compounds having Formula I:

X-L-P

or a salt form thereof, wherein X is a phytosterol, L is a linker, and P is water-soluble carrier moiety.

A phytosterol (X) refers to any of a group of sterols and stanols found naturally in plants, or partially or fully hydrogenated forms thereof (converting sterols to stanols) produced after removal from plants. Phytosterols can be derived from soft plants, e.g., soybeans, or alternatively from so-called "tall oil" extracted from woody plants, e.g., pine trees. Alternatively, the corresponding compounds can be produced synthetically. Preparations of phytosterols may include combinations of phytosterols from these different sources, and typically include mixtures of both sterols and stanols. The definition of phytosterols is intended to include any and all combinations of plant-derived sterols and stanols such as sitosterol and sitostanol (e.g., β-sitosterol and β-sitostanol), campesterol and campestanol, stigmasterol and stigmastanol, ergosterol and ergostanol, avenasterol and avenastanol, spinasterol and spinastanol, brassicasterol and brassicastanol, and clionasterol and clionastanol. The term also includes non-esterified phytosterols that have been partially or fully converted to non-esterified stanols, typically by chemical hydrogenation. In some embodiments, the phytosterol is a sitosterol such as β-sitosterol.

A linker (L), as used herein, preferably refers to a dicarboxylate moiety, such as, for example, -O₂C(CH₂)ₙCO₂- wherein n is an integer from 1 to 10 (e.g., 1 to 8, 1 to 6, 1 to 4, and 1 to 3). For example, a linker can be chosen from the group consisting of succinate, adipate, glutarate, pimelate, and malonate. In some embodiments, the linker is succinate. In coupling reactions, a linker may be present in the reaction as an acid, salt, or anhydride. For example, when the linker is succinate, succinic anhydride can be used in the coupling reactions.

A water-soluble carrier moiety is a hydrophilic molecule having an esterifiable hydroxy or carboxy group. The water-soluble carrier moiety is selected from the group consisting of polyethylene glycols, polyalcohols, and polyethers. The polyethylene glycols are those having an average molecular weight from about 200 to about 2000 (e.g., about 200 to about 1500; about 200 to about 1200; about 200 to about 1000; about 200 to about 800, about 200 to about 600, about 400 to about 1000, about 400 to about 800, about 400 to about 600; about 400 to about 1500; about 500 to about 1200; and about 800 to about 1200). In some embodiments, the polyethylene glycol has an average molecular weight of about 600. In other embodiments, the polyethylene glycol has an average molecular weight of about 1000.

As used herein, "about" is meant to account for variations due to standard experimental error (e.g., ± 10%).

The term "salt form" refers to salts which possess utility in various applications. For example, salts may possess properties such as high crystallinity, which may render them useful, for example in processes of synthesis, purification or formulation of compounds described herein. In general the useful properties of the compounds described herein do not depend critically on whether the compound is or is not in a salt form, so unless clearly indicated otherwise (such as specifying that the compound should be in "free base" or "free acid" form), reference in the specification to a compound of formula I should be understood as encompassing salt forms of the compound, whether or not this is explicitly stated. Salts may be prepared by conventional means from the corresponding compound by reacting, for example, the appropriate acid or base with a compound as described herein. A person skilled in the art will know how to prepare and select suitable salt forms for example, as described in Handbook of Pharmaceutical Salts: Properties, Selection, and Use By P. H. Stahl and C. G. Wermuth (Wiley-VCH 2002).

### Methods of making water-soluble phytosterol derivatives

A process for preparing water-soluble phytosterol derivatives (X-L-P) as described herein includes coupling reactions wherein coupling reagents are added to a mixture comprising a phytosterol intermediate (X-L) and a water-soluble carrier moiety (e.g., polyethylene glycol (PEG) with of an average molecular weight of about 200 to about 2,000) in a mole ratio of about 1:1.5 to 1:10 to prepare water-soluble phytosterol derivatives having a phytosterol intermediate attached to one side of a water-soluble carrier moiety.

In one embodiment, a method provided herein for the preparation of a water-soluble phytosterol derivative of Formula I (X-L-P), or a salt form thereof, can include reacting a mixture of a phytosterol intermediate of Formula II:

X-L

or a salt form thereof, wherein X is a phytosterol and L is a linker; a water-soluble carrier moiety (P); and an acid in a first organic solvent. The molar ratio of the phytosterol intermediate to water-soluble carrier moiety is from about 1:1.5 to about 1:10. Following this reaction, the resulting mixture is neutralized and a second organic solvent is added. The neutralized solution can be washed with brine and a composition having a water-soluble phytosterol derivative (X-L-P) can be isolated.

Reacting (e.g., coupling) the phytosterol intermediate and the water-soluble carrier moiety can include heating the reaction. For example, the reaction mixture can be heated to a temperature above the refluxing temperature of the first organic solvent for a time necessary to complete the coupling reaction.

An acid, as used herein, can be any acid capable of promoting the esterification of the water-soluble carrier moiety with a phytosterol intermediate. For example, acids can include sulfuric acid, p-toluene sulfonic acid, methanesulfonic acid, trichloroacetic acid, oxalic acid, hydrochloric acid, and the like. In some embodiments, the acid is a strong acid such as sulfuric acid. The acid can be present in any amount necessary to promote the coupling reaction. For example, the acid can be present in a catalytic amount.

In some cases, the organic solvents used herein can be independently selected from solvents that when used under good manufacturing practices are suitable for GRAS (Generally Recognized As Safe) approval. In some embodiments the solvents can be those considered to be GRAS (Generally Recognized As Safe) solvents, such as those meeting the descriptions provided in 21 C.F.R 170.3 and 21 C.F.R. 170.30. Examples of suitable organic solvents for use in the methods described herein include acetic acid, acetone, acetonitrile, anisole, 1-butanol, 2-butanol, butyl acetate, tert-butylmethyl ether, cumene, cyclohexane, 1,2-dicloroethene, dichloromethane, dimethyl sulfoxide, N,N-dimethylacetamide, N,N-dimethylformamide, ethanol, ethyl acetate, ethyl ether, ethyl formate, ethylene glycol, formic acid, heptane, isobutyl acetate, isopropyl acetate, methanol, methylcyclohexane, N-methylpyrrolidone, methyl acetate, 3-methyl-1-butanol, methylethyl ketone, methylisobutyl ketone, 2-methyl-1-propanol, pentane, 1-pentanol, 1-propanol, 2-propanol, propyl acetate, tetrahydrofuran, toluene, xylene and mixtures thereof. In some embodiments, the organic solvents can be selected from toluene and ethyl acetate. A first organic solvent, second organic solvent, and/or third organic solvent, as used herein, can be the same or different and can be composed of one or more organic solvents as described above.

The mole ratio of phytosterol intermediate to water-soluble carrier can range from about 1:1.5 to 1:10. For example, the ratio can be selected from about 1:1.5 to about 1:9; about 1:1.5 to about 1:8; about 1:1.5 to about 1:6; about 1.1.5 to about 1:4; about 1.15 to about 1:3, 1:2 to about 1:8, about 1:2 to about 1:6, about 1:2 to about 1:5, about 1:2 to about 1:4, about 1:2.5 to about 1:8, about 1:2.5 to about 1:6, about 1:2.5 to about 1:4, about 1:3 to about 1:9, about 1:3 to about 1:7, about 1:3 to about 1:6, about 1:4 to about 1:8, and about 1:5 to about 1:8. In some cases, the mole ratio of phytosterol intermediate to water-soluble carrier can be about 1:5. In some cases, the mole ratio of phytosterol intermediate to water-soluble carrier can be about 1:3. In some cases, the mole ratio of phytosterol intermediate to water-soluble carrier can be between about 1:1.5 to about 1:3. Controlling the mole ratio of these two components in the coupling reaction has been found to decrease the production of the undesired di-type phytosterol derivative, therefore increasing the concentration of mono-type derivatives in the final product.

The crude water-soluble phytosterol derivative is purified by washing the reaction product with a brine solution. As used herein, brine is an aqueous solution having at least 15% by weight of one or more alkaline metals (e.g., at least 20% by weight of one or more alkaline metals, at least 25% by weight of one or more alkaline metals, at least 30% by weight of one or more alkaline metals, at least 35% by weight of one or more alkaline metals, and at least 39% by weight of one or more alkaline metals). In some embodiments, a solution having at least 15% by weight sodium chloride can be used (e.g., at least 20% by weight sodium chloride, at least 25% by weight sodium chloride, at least 30% by weight sodium chloride, at least 35% by weight sodium chloride, and at least 39% by sodium chloride). For example, a solution having about 35% by weight sodium chloride can be used. In some embodiments, brine can be water saturated or nearly saturated with one or more salts of an alkaline metal (e.g., lithium, sodium, and potassium). For example, brine can be prepared using sodium chloride.

Without being bound by theory, purification of the water-soluble phytosterol derivative is thought to occur because polyethylene glycol is more soluble in brine than the water-soluble phytosterol derivative. This solubility difference facilitates expedient removal of unreacted polyethylene glycol from the desired water-soluble phytosterol derivative product. In some cases, this method of purifying a solution comprising a water-soluble phytosterol derivative can be employed on any composition comprising a water-soluble phytosterol derivative in need of further purification. For example, a solution comprising a water-soluble phytosterol derivative and an organic solvent can be washed with brine and the purified water-soluble phytosterol derivative can then be isolated from the organic solvent having had one or more impurities removed.

Isolating a water-soluble phytosterol derivative, phytosterol intermediate or a composition comprising the same can include any conventional isolation and/or purification method. The isolation method can include, for example, distillation, recrystallization, column chromatography, ion exchange chromatography, gel chromatography, affinity chromatography, preparative thin layer chromatography, extraction with a solvent, and the like. In some embodiments, a water-soluble phytosterol derivative can be isolated or purified through recrystallization from an organic solvent (e.g., ethyl acetate, methyl *tert*-butyl ether).

As a non-limiting example of the methods described above, a water-soluble phytosterol derivative according to Formula III: 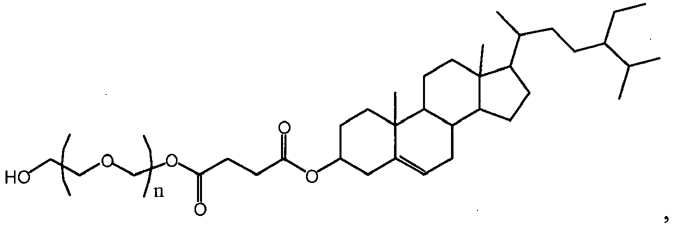 or a salt form thereof, can be prepared according to scheme I:

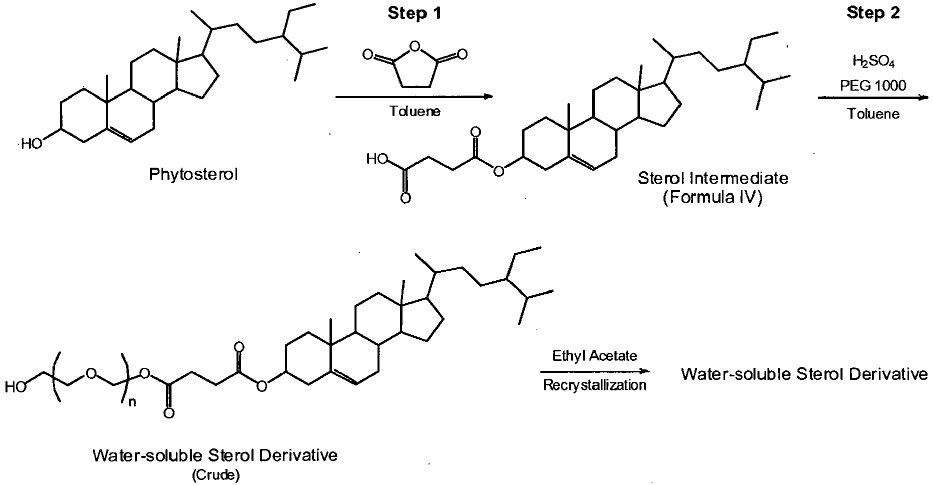

The water-soluble phytosterol derivative shown above can be prepared, for example, by combining three molar equivalents of polyethylene glycol with an average molecular weight of 1000 in a first organic solvent (e.g., toluene) with an acid (e.g., sulfuric acid) and heated to reflux. To this mixture one molar equivalent of phytosterol intermediate (e.g., β-sitosterol succinate) can be dissolved in the first organic solvent and added slowly to the reaction mixture over a period of one to five hours. When the reaction is completed (after one to ten hours), the reaction mixture can be cooled to room temperature and neutralized (e.g., through addition of a base such as potassium carbonate). The resulting mixture can be diluted with a second organic solvent (e.g., ethyl acetate) and washed one to six times with brine. The organic layer can then be concentrated under vacuum and the water-soluble phytosterol derivative recrystallized from an organic solvent (e.g., ethyl acetate) to provide the purified water-soluble phytosterol derivative.

The phytosterol intermediates described above (e.g., X-L or a compound of Formula II) can be prepared using methods known to one of skill in the art (see, e.g., Chung et al., J. Ind. Eng. Chem., 13(3): 367-372 (2007)).

In some embodiments, a phytosterol intermediate has a structure according to Formula II:

X-L

or a salt form thereof, wherein X is a phytosterol and L is a linker, and can be prepared by reacting a mixture of a phytosterol and a linker in an organic solvent. The reaction mixture can then be cooled and filtered. The filtrate is then washed with water, and the phytosterol intermediate can then be isolated from the organic layer. In some embodiments, the reaction is performed in the absence of base. In some embodiments, a base can be added to the initial reaction of the phytosterol and linker. For example, one or more of 4-dimethylaminopyridine (DMAP), N,N-diisopropylethylamine (DIPEA), pyridine, or triethylamine (TEA) can be used to promote the coupling reaction. In some embodiments, the base is present in a catalytic amount.

As an example, phytosterol can be slurred in an organic solvent. To this mixture, succinic anhydride can be added and the agitated mixture heated to reflux. When the reaction is complete, the mixture can be cooled to 4°C and the solids removed. The organic layer can then be separated, evaporated on a rotary evaporator and slurred with an organic solvent. The precipitate is then collected by filtration and rinsed with cold organic solvent. The resulting solid can then be purified as described above.

The phystosterol intermediate can be isolated from the reaction mixture and in some cases, can be purified using methods known in the art. In some embodiments, the phytosterol intermediate can be prepared and used directly (e.g., without isolation of the solid from the reaction mixture), for example, in the production of a water-soluble phytosterol derivative.

In some embodiments, the compound of Formula II can be a compound of Formula IV: 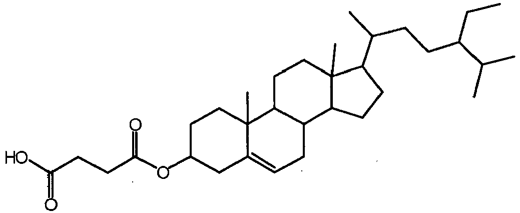 or a salt form thereof.

### Properties and methods of use

The water-soluble phytosterol derivatives prepared by the methods described herein can be soluble and/or dispersible in water in an amount of at least 30 mg by weight of phytosterol per mL of water and result in a clear solution. The solublity/dispersibility of these compounds is due in part to derivatives having a degree of substitution (DS) ranging from about 1.0 to about 1.3 (e.g., 1.0, 1.01, 1.02, 1.03, 1.04, 1.05, 1.06, 1.07, 1.08, 1.09, 1.1, 1.2, 1.22, 1.24, 1.25, 1.26, 1.28, and 1.3). In some embodiments, the DS can range from about 1.0 to about 1.15. As used herein, the term "degree of substitution" or DS, as it relates to water-soluble phytosterol derivatives, refers to the total number of phytosterol groups per water-soluble carrier (e.g., PEG) molecule.

The water-soluble phytosterol derivatives provided herein may be used as ingredients in food and as pharmaceuticals for their cholesterol-reducing effects. For example, the water-soluble phytosterol derivatives can be used to meet the effective daily dosage of phytosterols in adults in various types of products, such as cholesterol-lowering additives for food and beverages. Such products may be useful for the treatment of hypercholesterolemia and prevention of some cardiac diseases and hypertension.

Unless defined otherwise, all technical and scientific terms used herein have the same meaning as is commonly understood by one of ordinary skill in the art to which this disclosure pertains. All patents, applications, published applications, and other publications are incorporated by reference in their entirety. In the event that there is a plurality of definitions for a term herein, those in this section prevail unless stated otherwise.

### EXAMPLES

### Example 1: Preparation of water-soluble phytosterol derivative

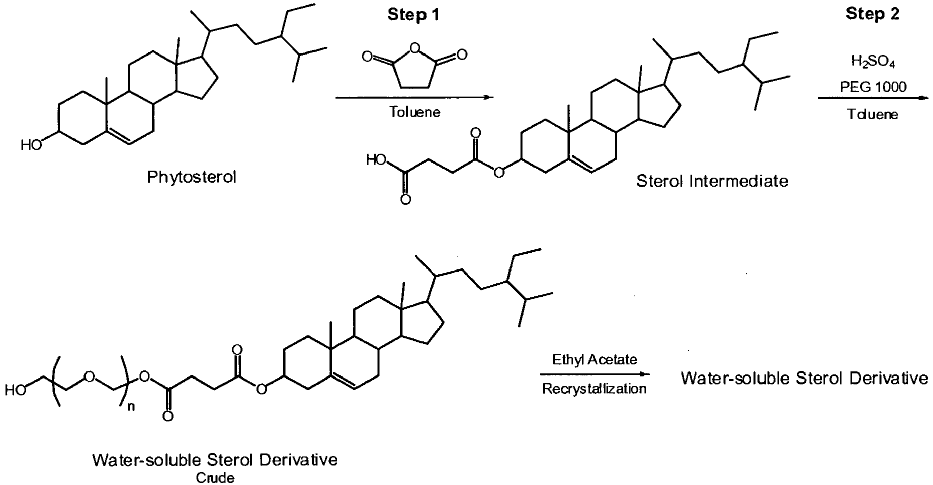

### Reparation of Phytosterol Intermediate (Step 1)

Phytosterol (100 g, 0.24 mol) was slurred in dry toluene and concentrated on a rotary evaporator to remove any residual water present. The phytosterol in toluene (400 mL) and succinic anhydride (32.6 g, 1.3 eq.) were combined and the agitated mixture was heated to 111°C. Progress of the reaction was monitored by TLC/NMR. When the reaction was complete, the mixture was cooled to 4°C and the solids were filtered off (6.4 g) using a Buchner funnel, PP filter cloth and filtration flask. The filtrate was diluted with MTBE and washed with water. The organic layer was evaporated on a rotary evaporator and slurred with heptane. The precipitate was collected by filtration and rinsed with cold heptane. The white solid was dried in a vacuum oven to give 104.4 g (84.5%) of phytosterol intermediate.

### Preparation of Water-soluble Phytosterol Derivative (Step 2)

PEG 1000 (292 g, 0.2928 mol, 5 eq.) in toluene (750 mL) and sulfuric acid (0.69 g) was heated to a gentle reflux. The Phytosterol Intermediate of step 1 (30.1 g, 0.0585 mol, 1.0 eq.) was dissolved in toluene (140 mL) and added slowly to the reaction mixture (over a period of 3 hrs). The reaction progress was monitored by TLC. When the reaction was complete (about 4 hours), it was cooled to room temperature and solid potassium carbonate (∼1 g) was added. The mixture was diluted with ethyl acetate (1400 mL) and washed with brine (5 × 1400 mL). The organic layer was concentrated under vacuum to a light yellow solid (78.1 g) which showed a Degree of Substitution (DS) of 1.19. The solid was dissolved in ethyl acetate (55 mL) at 40°C then chilled to 5°C. The precipitated solid was collected by filtration and washed with cold ethyl acetate (20 mL). The off-white solid was dried in a vacuum oven to yield 46.9 g (53%) of water-soluble phytosterol derivative with a DS of 1.06. The structure of the compound was confirmed by ¹H NMR.

### Preparative Example 2: Reparation of phytosterol intermediates

Phytosterol (1 eq.), succinic anhydride (1.5 eq.), toluene (4 eq.), and a diisopropylethyl amine catalyst (0.2%) were combined and heated to 100°C for three hours. The resulting solution was cooled to 4°C and filtered. Methyl *tert*-butyl ether (MTBE) was added (1:1) to the filtrate and the solution washed with water. The organic layer was then dried over MgSO₄, filtered, and concentrated to produce the phytosterol intermediate (90.3%).

### Preparative Example 3: Preparation of phytosterol intermediates

Phytosterol (1 eq.), succinic anhydride (1.3 eq.), toluene (4 eq.), and 4-dimethylaminopyridine (0.5%) were combined and heated to 100°C overnight. Methyl *tert-butyl* ether (MTBE) was added (1:1) and the solution washed with water. The organic layer was then dried over MgSO₄ and filtered. The filtrate was concentrated under reduced pressure and slurred with heptane prior to filtration to isolate the phytosterol intermediate.

### Preparative Example 4: Preparation of phytosterol intermediates

Phytosterol (5 g), succinic anhydride (1.5 eq.) and toluene (4 eq.) were combined and heated to 110°C overnight. The resulting solution was cooled to 4°C and filtered. Methyl *tert-butyl* ether (MTBE) was added (1:1) to the filtrate and the solution washed with water. The organic layer was then dried over MgSO₄, filtered, and concentrated to produce the phytosterol intermediate (73%).

### Comparative Example 5: Preparation of water-soluble phytosterol derivatives

PEG 1000 (5 eq.), β-sitosterol succinate (1 eq.) and sulfuric acid (catalyst) were combined with toluene (approx. 30 eq.) and heated to reflux for 3 hours and then cooled. Solid sodium potassium carbonate was added to neutralized the solution and the resulting mixture was filtered. Various work-up procedures were then implemented.
a) The filtrate was cooled to 4°C and allowed to warm-up. This procedure resulted in a product having a DS of 0.45.
b) The filtrate was cooled to 4°C and filtered ice cold; producing a product having a DS of 1.04.
c) 25% heptanes was added to the filtrate. An oil formed and was removed leaving a waxy solid having a DS of 0.53. Part of this waxy solid was run through a silica plug filter with 5% methanol in ethyl acetate. The desired product was the first fraction and gave a product having a DS of 0.53. The remainder of the waxy solid was dissolved in toluene. 15% water was added to the resulting solution and the mixture filtered. A product was isolated having a DS of 1.04.

### Comparative Example 6: Preparation of water-soluble phytosterol derivatives

PEG 1000 (3 eq.), β-sitosterol succinate (1 eq.) and sulfuric acid (catalyst) were combined with toluene (approx. 30 eq.) and heated to reflux for 3 hours and then cooled. Solid sodium potassium carbonate was added to neutralized the solution and the resulting mixture was filtered. Various work-up procedures were then implemented.
a) 15% water was added to the filtrate, but it was determined that the resulting solid could not be removed by filtration.
b) 1:1 toluene:heptanes was used to wash the filtrate in a separation funnel. The resulting oil layer was removed. Water was added to part of the remaining solution which was then filtered cold and the organic layer reduced. The resulting product had a DS of 1.96. The remaining portion of the solution was allowed to warm up and then was filtered. The resulting product had a DS of 0.98.

### Comparative Example 7: Preparation of water-soluble phytosterol derivatives

PEG 1000 (3 eq.) and sulfuric acid (catalyst) were combined with toluene (approx. 30 eq.) and heated to reflux. β-sitosterol succinate (1 eq.) was dissolved in toluene and slowly added to the refluxing reaction mixture (addition 30 minutes on and 30 minutes off for 2.5 hours). The reaction completed 1 hour after addition. Various work-up procedures were then implemented.
a) 1:1 toluene:heptanes was added to the solution and the mixture was allowed to settle for 1 hour. The resulting oil layer was removed. A portion of the organic layer was removed and filtered, producing a product having a DS of 0.76, To the remaining organic solution, the ratio of toluene to heptanes was raised to 1:3, but the resulting solution was still cloudy after filtering. The resulting product had a DS of 1.02.
b) The mixture was allowed to sit for at least 48 hours. After this time the solution was clear. Heptanes was added to raise the ratio of toluene:heptanes to 1:3 resulting in a cloudy mixture. Part of the mixture was vacuum filtered resulting in a cloudy filtrate and a product having a DS of 1.49. The remaining portion of the mixture was gravity filtered and produced a clear solution. The resulting product had a DS of 2.25.

### Comparative Example 8: Preparation of water-soluble phytosterol derivatives

PEG 1000 (3 eq.) and sulfuric acid (catalyst) were combined with toluene (approx. 20 eq.) and heated to reflux. β-sitosterol succinate (1 eq.) was dissolved in toluene and slowly added to the refluxing reaction mixture (addition 30 minutes on and 30 minutes off for 2.5 hours). The reaction completed 1 hour after addition. Various work-up procedures were then implemented.
a) 0.5:1 heptanes:toluene was added. The product isolated from the resulting filtrate had a DS of 0.88.
b) 1:1 heptanes:toluene was added. The product isolated from the resulting filtrate had a DS of 0.87.
c) 2:1 heptanes:toluene was added. The product isolated from the resulting filtrate had a DS of 1.3.
d) 3:1 heptanes:toluene was added. The product isolated from the resulting filtrate had a DS of 2.3.
e) The solid from work-up a) was dissolved in dichloromethane and washed with brine. The resulting filtrate had a DS of 0.88.
f) The solid from work-up a) was titrated with MTBE. The resulting filtrate had a DS of 1.08, but a very low yield.
g) The solid from work-up a) was titrated with 90:10 MTBE:toluene. The resulting filtrate had a DS of 1.05 and a 30% yield.
h) The solvent was removed from the reaction mixture and the solid was dissolved in toluene. To this solution 1 part MTBE was added. This solution was washed with 1 equivalent of brine (4 times), once with 15% NaCl, and then again washed with brine. The organic layer was then dried with MgSO₄ and filtered. The product isolated from the filtrate had a DS of 2.6.
i) The solid from work-ups c) and d) were dissolved in 6 equivalents of toluene. To this 5 equivalents of heptanes were added. The solution was allowed to crystallize and the resulting crystals had a DS of 1.01.

### Comparative Example 9: Preparation of water soluble phytosterol derivatives

PEG 1000 (3 eq.) and sulfuric acid (catalyst) were combined with toluene (150 mL) and heated to reflux. β-sitosterol succinate (1 eq.) was dissolved in toluene and slowly added to the refluxing reaction mixture (addition 30 minutes on and 30 minutes off for 2.5 hours). The reaction completed 1 hour after addition. The reaction mixture was diluted with 1 equivalent of dichloromethane and washed with a saturated brine solution (6 times). The resulting product had a DS of 0.51 indicating at least 1 equivalent of unreacted PEG. Half of this solid was redissolved in toluene/DCM (100 mL, 1:1 v/v) and washed with 24% brine (6 times). The resulting product had a DS of 1.16. The other half of the solid was redissolved in toluene/ethyl acetate and washed with 36% brine (3 times) and gave a product having a DS of 1.2. Further washing with brine did not change the DS (1.22) indicating that all unreacted PEG had been removed with the previous washings.

### Example 10: Preparation of water-soluble phytosterol derivatives

A 15 g scale reaction was completed using PEG 1000 (3 eq.), sulfuric acid (catalyst), and toluene (approx. 20 eq.) heated to reflux. β-sitosterol succinate (1 eq.) was dissolved in toluene and added slowly to the refluxing mixture over 1.5 hours. The reaction was deemed completed 2 hours following addition of the β-sitosterol succinate. After base was added to neutralize the reaction mixture, the solution was cooled and ethyl acetate was added (1 eq.). This solution was washed with brine (1.0 eq.) eight times. The organic layer was reduced to a yellow oil (38.1 g, 85% yield), with a DS of 1.13. The oil was then dissolved into 1 gram of isopropanol and chilled for 48 hours. A slightly yellow material (0.6 g) with a DS of 1.13 was isolated.

### Example 11: Preparation of water-soluble phytosterol derivatives

A 30 g scale reaction was completed using PEG 1000 (5 eq.), sulfuric acid (catalyst), and toluene (approx. 30 eq.) heated to reflux. β-sitosterol succinate (1 eq.) was dissolved in toluene and added slowly (two additions with a 45 minute break between) to the refluxing mixture over 4.5 hours. The reaction was deemed completed 4 hours following addition of the β-sitosterol succinate. After base was added to neutralize the reaction mixture, the solution was cooled and ethyl acetate was added (1 eq.). This solution was washed with brine (1.0 eq.) five times. The organic layer was reduced to a yellow oil (78.1 g, 88% yield), with a DS of 1.19. The oil was then dissolved into 55 mL of ethyl acetate and chilled at 4°C overnight. The resulting solid was filtered with the addition of 20 mL cold ethyl acetate. The off-while solid produced (50.8 g, 58% yield) had a DS of 1.06.

## Claims

1. A method of making a compound of Formula I:
X-L-P
or a salt form thereof, wherein:
X is a phytosterol;
L is a linker; and
P is a water-soluble carrier selected from the group consisting of polyethylene glycol having an average molecular weight of about 200 to about 2000, polyalcohol, and polyether; said method comprising:
(a) reacting a mixture comprising X-L, P, an acid, and a first organic solvent, wherein the molar ratio of X-L to P is from about 1:1.5 to about 1:10;
(b) neutralizing said mixture of step (a) and adding a second organic solvent;
(c) washing said solution from step (b) with brine; and
(d) isolating a composition comprising said compound of Formula I,
wherein said compound of Formula I has a degree of substitution of 1.0 to about 1.3.

2. The method of claim 1, wherein said method further comprises recrystallizing the compound of Formula I.

3. The method of claim 1, wherein said molar ratio of X-L to P is from about 1:2 to about 1:6.

4. The method of claim 1, wherein said phytosterol is selected from the group consisting of:
sitosterol, sitostanol, campesterol, campestanol, stigmasterol, stigmastanol, ergosterol, ergostanol, avenasterol, avenastanol, spinasterol, spinastanol, brassicasterol, brassicastanol, clionasterol, clionastanol and mixtures thereof.

5. The method of claim 1, wherein said linker is a dicarboxylate.

6. The method of claim 1, wherein said P is polyethylene glycol having an average molecular weight of about 200 to about 2000.

7. The method of claim 1, wherein said acid is selected from the group consisting of sulfuric acid, *p*-toluene sulfonic acid, and methanesulfonic acid.

8. The method of claim 1, wherein said first and second organic solvents are independently selected from GRAS solvent wherein gras stands for: generally recognised as safe

9. The method of claim 1, wherein said first solvent is toluene and said second solvent is ethyl acetate.

10. The method of claim 1, wherein said reacting comprises heating.

11. The method of claim 1, wherein said compound X-L, or a salt form thereof, is prepared by a method comprising:
(a) reacting a mixture of X and L in an organic solvent;
(b) cooling said reaction mixture of step (a);
(c) filtering said cooled reaction mixture of step (b); and
(d) washing said filtrate with water and isolating a composition comprising said compound X-L.

12. The method of claim 11, wherein said mixture of step (a) further comprises a base.

13. The method of claim 12, wherein said base is selected from the group consisting of DMAP, DIPEA, pyridine, and TEA.

14. The method of claim 1, wherein said composition comprising said compound of Formula I is soluble and/or dispersible in water in an amount of at least 30 mg of phytosterol per mL of water and forms a clear dispersion.

15. The method of claim 1, wherein said composition comprising said compound of Formula I has a degree of substitution of about 1.0 to about 1.15.

## Patentansprüche

1. Ein Verfahren zur Herstellung der Verbindung von Formel I:
X-L-P
oder ein Salz davon, wobei:
X ein Phytosterol ist;
L ein Linker ist; und
P ein wasserlöslicher Träger ist, ausgewählt aus der Gruppe, bestehend aus Polyethylenglykol mit einem durchschnittlichen Molekulargewicht von etwa 200 bis etwa 2000, Polyalkohol und Polyether; jenes Verfahren umfassend:
(a) Reaktion einer Mischung umfassend X-L, P, einer Säure und ein erstes organisches Lösungsmittel, wobei das molare Verhältnis von X-L zu P von etwa 1:1,5 bis etwa 1:10 ist;
(b) Neutralisieren jener Mischung aus Schritt (a) und Zugeben eines zweiten organischen Lösungsmittels;
(c) Waschen jener Lösung aus Schritt (b) mit Salzlösung; und
(d) Isolieren einer Zusammensetzung umfassend jene Verbindung von Formel I, wobei jene Verbindung von Formel I einen Substitutionsgrad von 1,0 bis etwa 1,3 hat.

2. Das Verfahren nach Anspruch 1 wobei jenes Verfahren weiter Rekristallisieren der Verbindung von Formel I umfasst.

3. Das Verfahren nach Anspruch 1, wobei jenes molare Verhältnis von X-L zu P von etwa 1:2 bis etwa 1:6 ist.

4. Das Verfahren von Anspruch 1, wobei jenes Phytosterol ausgewählt ist aus der Gruppe, bestehend aus: Sitosterol, Sitostanol, Campesterol, Campestanol, Stigmasterol, Stigmastanol, Ergosterol, Ergostanol, Avenasterol, Avenastanol, Spinasterol, Spinastanol, Brassicasterol, Brassicastanol, Clionasterol, Clionastanol und Mischungen davon.

5. Das Verfahren nach Anspruch 1, wobei jener Linker ein Dicarboxylat ist.

6. Das Verfahren nach Anspruch 1, wobei jenes P Polyethylenglykol mit einem durchschnittlichen Molekulargewicht von etwa 200 bis etwa 2000 ist.

7. Das Verfahren nach Anspruch 1, wobei jene Säure ausgewählt ist aus der Gruppe bestehend aus Schwefelsäure, *p*-Toluolsulfonsäure und Methansulfonsäure.

8. Das Verfahren nach Anspruch 1, wobei jenes erste und zweite organische Lösungsmittel unabhängig ausgewählt sind von GRAS-Lösungsmitteln, wobei GRAS steht für:
Generally Recognised as Safe (Allgemein als sicher anerkannt).

9. Das Verfahren nach Anspruch 1, wobei das erste Lösungsmittel Toluol und das zweite Lösungsmittel Ethylacetat ist.

10. Das Verfahren nach Anspruch 1, wobei jene Reaktion Erhitzen umfasst.

11. Das Verfahren von Anspruch 1, wobei jene Verbindung X-L oder ein Salz davon durch ein Verfahren aufbereitet wird umfassend:
(a) Reaktion einer Mischung von X und L in einem organischen Lösungsmittel;
(b) Kühlen jener Reaktionsmischung aus Schritt (a);
(c) Filtrieren jener Reaktionsmischung aus Schritt (b); und
(d) Waschen jenes Filtrats mit Wasser und Isolieren einer Zusammensetzung umfassend jene Verbindung X-L.

12. Das Verfahren nach Anspruch 11, wobei jene Mischung aus Schritt (a) weiter eine Base umfasst.

13. Das Verfahren nach Anspruch 12, wobei jene Base ausgewählt ist aus der Gruppe bestehend aus DMAP, DIPEA, Pyridin und TEA.

14. Das Verfahren nach Anspruch 1, wobei jene Zusammensetzung, die jene Verbindung von Formel I umfasst, wasserlöslich und/oder in Wasser dispergierbar ist in einer Menge von mindestens 30 mg Phytosterol pro ml Wasser und eine klare Dispersion bildet.

15. Das Verfahren von Anspruch 1, wobei jene Zusammensetzung, die jene Verbindung von Formel I umfasst, einen Substitutionsgrad von etwa 1,0 bis etwa 1,15 hat.

## Revendications

1. Procédé de fabrication d'un composé de formule I :
X-L-P
ou d'une forme de sel de celui-ci, dans laquelle :
X est un phytostérol ;
L est un lieur ; et
P est un véhicule soluble dans l'eau sélectionné dans le groupe constitué du polyéthylène glycol ayant une masse moléculaire moyenne en poids d'environ 200 à environ 2000, d'un polyalcool, et d'un polyéther ; ledit procédé comprenant :
(a) la réaction d'un mélange comprenant X-L, P, un acide, et un premier solvant organique, dans lequel le rapport molaire de X-L sur P est d'environ 1/1,5 à environ 1/10 ;
(b) la neutralisation dudit mélange de l'étape (a) et l'ajout d'un second solvant organique ;
(c) le lavage de ladite solution de l'étape (b) avec de la saumure ; et
(d) l'isolement d'une composition comprenant ledit composé de formule I,
dans lequel ledit composé de formule I a un degré de substitution de 1,0 à environ 1,3.

2. Procédé selon la revendication 1, dans lequel ledit procédé comprend en outre la recristallisation du composé de formule I.

3. Procédé selon la revendication 1, dans lequel ledit rapport molaire de X-L sur P est d'environ 1/2 à environ 1/6.

4. Procédé selon la revendication 1, dans lequel ledit phytostérol est sélectionné dans le groupe constitué : du sitostérol, du sitostanol, du campestérol, du campestanol, du stigmastérol, du stigmastanol, de l'ergostérol, de l'ergostanol, de l'avénastérol, de l'avénastanol, du spinastérol, du spinastanol, du brassicastérol, du brassicastanol, du clionastérol, du clionastanol et de leurs mélanges.

5. Procédé selon la revendication 1, dans lequel ledit lieur est un dicarboxylate.

6. Procédé selon la revendication 1, dans lequel ledit P est le polyéthylène glycol ayant une masse moléculaire moyenne d'environ 200 à environ 2000.

7. Procédé selon la revendication 1, dans lequel ledit acide est sélectionné dans le groupe constitué de l'acide sulfurique, de l'acide p-toluène sulfonique, et de l'acide méthanesulfonique.

8. Procédé selon la revendication 1, dans lequel lesdits premier et second solvants organiques sont indépendamment sélectionnés parmi les solvants dits GRAS, dans lequel GRAS signifie : Generally Recognised as Safe (généralement reconnus comme sûrs).

9. Procédé selon la revendication 1, dans lequel ledit premier solvant est le toluène et ledit second solvant est l'acétate d'éthyle.

10. Procédé selon la revendication 1, dans lequel ladite réaction comprend le chauffage.

11. Procédé selon la revendication 1, dans lequel ledit composé X-L, ou une forme de sel de celui-ci, est préparé par un procédé comprenant :
(a) la réaction de X et L dans un solvant organique ;
(b) le refroidissement dudit mélange réactionnel de l'étape (a) ;
(c) la filtration dudit mélange réactionnel refroidi de l'étape (b) ; et
(d) le lavage dudit filtrat avec de l'eau et l'isolement d'une composition comprenant ledit composé X-L.

12. Procédé selon la revendication 11, dans lequel ledit mélange de l'étape (a) comprend en outre une base.

13. Procédé selon la revendication 12, dans lequel ladite base est sélectionnée dans le groupe constitué de la DMAP, de la DIPEA, de la pyridine, et de la TEA.

14. Procédé selon la revendication 1, dans lequel ladite composition comprenant ledit composé de formule I est soluble et/ou dispersible dans l'eau en une quantité d'au moins 30 mg de phytostérol par mL d'eau et forme une dispersion claire.

15. Procédé selon la revendication 1, dans lequel ladite composition comprenant ledit composé de formule I a un degré de substitution d'environ 1,0 à environ 1,15.
